Europäisches Patentamt

European Patent Office

Office européen des brevets

(11) Numéro de publication: **0 206 842**
**A1**

(12) # DEMANDE DE BREVET EUROPEEN

(21) Numéro de dépôt: **86400797.6**

(22) Date de dépôt: **14.04.86**

(51) Int. Cl.4: **C07K 15/00** , **C12P 21/00** ,
**C12N 5/00** , **C12N 15/00** ,
**C12N 7/00** , **C07K 3/18** ,
**G01N 33/569** , **A61K 39/21** ,
**//(C12P21/00,C12R1:91)**

(30) Priorité: **15.04.85 FR 8505676**

(43) Date de publication de la demande:
**30.12.86 Bulletin 86/52**

(84) Etats contractants désignés:
**AT BE CH DE FR GB IT LI LU NL SE**

(71) Demandeur: **INSTITUT PASTEUR**
**25-28, rue du Docteur Roux**
**F-75724 Paris Cédex 15(FR)**
Demandeur: Etablissement Public dit:
**CENTRE NATIONAL DE LA RECHERCHE**
**SCIENTIFIQUE (CNRS)**
**15, Quai Anatole France**
**F-75700 Paris(FR)**

(72) Inventeur: **Montagnier, Luc**
**21 rue de Malabry**
**F-92350 Le Plessis Robinson(FR)**
Inventeur: **Rey, Françoise**
**10 rue d'Odessa**
**F-75014 Paris(FR)**
Inventeur: **Krust, Bernard**
**7 rue de Madagascar**
**F-75012 Paris(FR)**
Inventeur: **Clavel, François**
**83 rue de l'Assomption**
**F-75016 Paris(FR)**

(74) Mandataire: **Gutmann, Ernest et al**
**S.C. Ernest Gutmann - Yves Plasseraud 67,**
**boulevard Haussmann**
**F-75008 Paris(FR)**

(54) Antigènes apparentés à la glycoprotéine d'enveloppe du virus du syndrome d'immunodéficience acquise.

(57) Demande de premier certificat d'addition à la demande de brevet n° 84 16013 déposée le 18 octobre 1984.

L'invention concerne des antigènes précurseurs de la glycoprotéine ayant un poids moléculaire de l'ordre de 110.000 appartenant à l'enveloppe du virus du SIDA. Cet antigène peut être détecté par marquage du virus avec une concentration élevée de $^{35}$S-cystéine. L'invention concerne plus particulièrement encore un procédé mettant en jeu lesdits antigènes précurseurs de diagnostic de la présence d'anticorps contre l'agent étiologique de SIDA dans le sérum de patients affectés par le SIDA ou le LAS ou de porteurs asymptomatiques de virus.

Elle concerne enfin des compositions immunogènes contenant l'antigène du genre en question en tant que principe immunogène.

# Antigènes apparentés à la glycoprotéine d'enveloppe du virus de SIDA, notamment précurseurs de cette glycoprotéine, procédés d'obtention de ces antigènes et moyens mis en oeuvre dans ces procédés, applications de ces antigènes à la préparation de compositions immunogènes ou pour le diagnostic du SIDA ou des affections qui lui sont apparentées.

La présente invention est relative à des antigènes apparentés à glycoproéine d'enveloppe du virus de SIDA, notamment à des précurseurs de cette glycoprotéine et à des fragments dérivés de ces antigènes. Elle est encore relative à des moyens pour l'obtention de ces antigènes, précurseurs et fragments, ainsi qu'aux applications de ces antigènes, précurseurs et fragments à la production de compositions immunogènes ou encore à de compositions utiles pour le diagnostic du SIDA ou des affections qui lui sont apparentées, telles que des lymphadénopathies (LAS) ou plus généralement encore de la présence dans un milieu biologique, tel qu'un sérum humain, d'anticorps aptes à sélectivement reconnaître le virus lav ou les virus qui lui sont apparentés. Le préambule de la demande de brevet n° 84 16013 déposée le 18 octobre 1984, et à laquelle se rattache la présente demande de certificat d'addition, a rappelé un certain nombre de références bibliographiques d'articles scientifiques dans lesquels de tels virus apparentés ont été décrits.

La demande de brevet principal a déjà décrit une glycoprotéine d'enveloppe ayant un poids moléculaire d'environ 110.000 daltons, glycoprotéine ci-après désignée sous l'abréviation "gp110", plus particulièrement de poids moléculaire apparent de 110.000-120.000, dont la présence peut être détectée dans des lysats de virus LAV obtenus à partir de lymphocytes infectés et marqués par la $^{35}$S-cystéine. Cette glycoprotéine est sélectivement reconnue par des sérums provenant de sujets atteints de LAS ou de SIDA.

La présente invention a pour objets différents antigènes apparentés à la gp110, soit qu'ils s'agissent de précurseurs, soit qu'ils s'agissent de fragments de la gp110, et de nouveaux moyens - (souches cellulaires et procédés) permettant d'obtenir tous ces antigènes, y inclus la gp110.

A ce titre l'invention concerne plus particulièrement des glycoprotéines présentant des poids moléculaires de l'ordre de 150.000 et 135.000 daltons (poids moléculaires appréciés par mesure des distances de migration de ces glycoprotéines, par comparaison avec celles de protéines de référence et de poids moléculaires connus, telles que celles qui ont été identifiées dans le brevet principal), ces nouvelles glycoprotéines présentant également des propriétés caractéristiques de la gp110 :

-capacité d'être marquées par la $^{35}$S-cystéine,

-capacité de former des complexes avec la concanavaline A, ces complexes pouvant ensuite être dissociés en présence de O-méthyl-alpha-D-mannopyranoside,

-capacité de former des complexes avec d'autres lectines, telles que celles connues sous la désignation "LENTYL-LECTINES",

-sensibilité à l'action d'endoglycosidases, particulièrement de l'endo-bêta-méthylglycosaminidase-H (sous forme abrégée : endo-H),

-capacité d'être marquées par la [$^{14}$C]-glucosamine,

-capacité d'être reconnues spécifiquement par des sérums de personnes affectées par les SIDA ou LAS.

Dans ce qui suit les glycoprotéines de poids moléculaires 150.000 et 135.000 daltons seront respectivement désignées sous les abréviations "gp150" et "gp135".

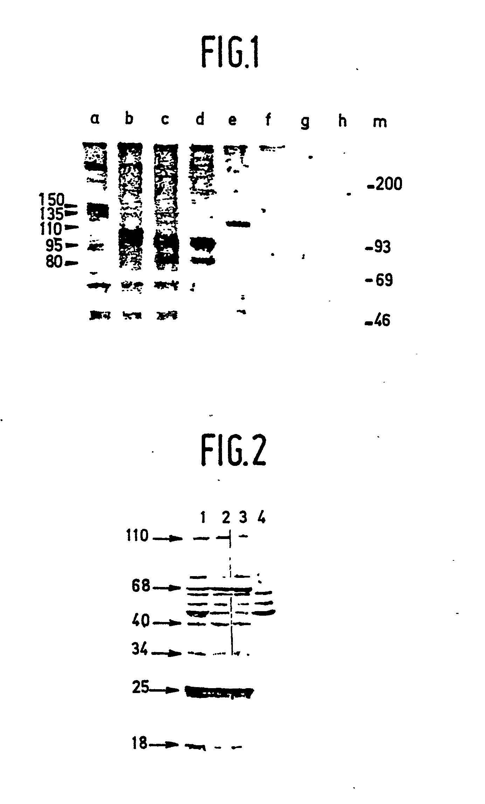

La structure de l'ossature polypeptidique de la gp150 semble devoir être corrélée à celle résultant de la capacité codante totale du gène "env", dont la structure nucléotidique peut être déduite de l'article de WAIN-HOBSON, S. et al, Cell, vol. 40, p. 9-17, 1985. Cette ossature polypeptidique (poids moléculaire apparent de l'ordre de 90.000) paraît correspondre à la séquence polypeptidique codée par la séquence nucléotidique s'étendant entre la position 5767 et la position 8350, telle qu'elle résulte respectivement de la structure nucléotidique de la fig. 1 de l'article de WAIN-HOBSON.

A la gp135 paraît devoir correspondre une ossature polypeptidique plus courte, dérivée de la précédente, à la suite d'un clivage protéolytique d'une séquence peptidique terminale ayant un poids moléculaire d'environ 15.000, qui paraît correspondre à la partie C-terminale de l'ossature peptidique de la gp150.

L'invention concerne également des moyens permettant de produire des quantités importantes de ces précurseurs, en particulier des hybrides cellulaires formés entre des lymphocytes T4 normaux et des cellules de la lignée MOLT-4 décrite par MINOWADA, J. et al, J. Natl. Cancer Inst. 49 , 891 (1972) ou analogue, lesdits hybrides cellulaires étant caractérisés par leur capacité à exprimer la molécule T4 à leur surface et par leur susceptibilité à l'infection par le virus LAV ou par des virus apparentés.

Il est rappelé que des cellules de la lignée MOLT-4 sont dérivées de cellules de type lymphoïde qui ont été isolées à partir d'un malade atteint de leucémie.

Les hybrides du genre en question sont rendus capables de produire à titre principal les protéines codées par le génome viral, comme suite à leur infection par un virus LAV, d'où la possibilité d'obtenir des quantités appréciables desdites protéines et d'étudier l'évolution des proportions relatives de chacune d'elles au cours de cycle viral, comme cela résultera de la description donnée plus loin des conditions dans lesquelles de telles études peuvent être menées.

Ces hybrides peuvent notamment être fabriqués comme suit :

Des lymphocytes T4 d'un donneur de sang sont séparés selon la technique dcrite par KLATZMANN, D. et al, Nature (London), 312, 767 (1984). Ils sont ensuite fusionnés avec des cellules d'une lignée MOLT-4/TK⁻ en présence de polyéthylèneglycol selon les techniques désormais classiques pour la production d'hybridomes. Les hybrides formés sont sélectionnés, en milieu HAT dans les puits d'une plaque de microtitrage et l'on recueille ceux des clones qui expriment la molécule T4 à leur surface. Ces derniers clones peuvent être plus particulièrement reconnus par des anticorps monoclonaux commercialisés par la société ORTHO, sous la désignation commerciale OKT4.

Une lignée de cellules MOLT4/TK⁻, à partir desquelles les susdits hybrides cellulaires peuvent être formés, a fait l'objet d'un dépôt à la Collection Nationale de Culture de Micro-Organismes - (C.N.C.M.) de l'INSTITUT PASTEUR de Paris, le 15 avril 1985 sous le n° I-434.

Il va de soi que l'on peut utiliser à la place des lignées MOLT-4/TK⁻, tout autre mutant de MOLT-4 présentant une déficience génétique susceptible d'être complémentée par les lymphocytes T4 au cours de la fusion cellulaire, cette complémentation fournissant alors la base adéquate pour la sélection des hybrides recherchés.

Un hybride représentatif des hybrides selon l'invention a également fait l'objet d'un dépôt à la C.N.C.M., le 15 avril 1985, sous le n° I-435. De tels hybrides peuvent être cultivés dans le milieu décrit par MINOWATA, J. et al déjà cités.

Les hybrides cellulaires sélectionnés, notamment comme cela a été décrit plus haut, peuvent alors, après concentration par centrifugation, être infectés par un virus LAV ou analogue selon la technique rappelée dans la demande de brevet principal, notamment par remise en suspension de ces hybrides cellulaires dans le surnageant viral infextieux produit par des lymphocytes normaux préalablement infectés par un isolat de LAV obtenu à partir d'un malade atteint de LAS ou de SIDA, dans les conditions qui ont été décrites dans l'article de F. BARRE-SINOUSSI et al, Science, 220, 868 (1983) ou dans la demande de brevet européen déposée le 15 septembre 1984 sous le n° 84 401834, sous priorité de la demande britannique n° 83 24800 déposée le 15 setembre 1983.

Après infection par un virus LAV, tel que celui référencé sous la désignation LAV1 dans la susdite demande de brevet européen, les cellules d'hybride conduisent à la production de polycarions ou syncitias géants résultant de fusions entre des cellules hybrides infectées ou entre des cellules hybrides respectivement non infectées et infectées. Ces syncitias géants donnent lieu, notamment après fixation par l'acétone, à de fortes réactions d'immunofluorescence dans des essais d'imunofluorescence indirecte, avec du sérum provenant de porteurs d'anticorps anti-LAV. Les syncitias peuvent être aisément isolés, notamment par simple décantation sous l'effet de la gravité dans le milieu de culture ou dans le milieu dans lequel ils ont pu être remis en suspension.

La technique rappelée ci-dessus a permis, dans les conditions qui seront rappelées ci-après, de constater les moments du cycle viral où apparaissent lesdits précurseurs, lesquels peuvent être isolés dans les conditions décrites plus loin dans la légende relative à la fig. 1.

Il sera en particulier montré que la gp150 apparaît comme bande unique dans les premières heures de l'infection virale, par exemple au bout de 3 heures, tandis que la gp135 apparaît plus tard, notamment au bout de 12 heures.

Les conditions dans lesquelles apparaît la gp110 elle-même, et sa relation à d'autres antigènes susceptibles d'être isolés, seront encore décrites plus loin, et ce notamment en rapport avec la fig. 2, dont les légendes apparaissent également plus loin.

On décrit donc ci-après les conditions dans lesquelles la gp150 et la gp135 ont été détectées à partir de syncitias infectées dans les conditions sus-indiquées.

Après marquage pour des périodes de 3 ou de 12 heures, les syncitias ont été lysées dans une solution détergente et le lysat clarifié a été immunoprécipité avec un sérum LAV-positif, dénaturé, puis analysé par électrophorèse sur gel au dodécylsulfate de sodium (SDS). Après 3 heures de marquage, une bande de poids moléculaire 150.000 (150K) a été détectée. Après un marquage plus long (pendant 12 heures), une autre bande de 135K devait apparaître, celle-ci pouvant être considérée comme dérivée du précurseur 150K. Aucune bande de 110K-120K n'a été détectée, ce fait confirmant que la gp110 est associée avec les particules virales libres et non avec le virus en cours de formation.

Après traitement par l'endo-H, les bandes de 150K et 135K ont été réduites à deux bandes ayant respectivement des poids moléculaires de 95.000 - (95K) et 80.000 (80K), respectivement (fig. 1). Les hypothèses qui suivent peuvent d'ores et déjà être formulées, sous réserve d'un complément d'étude : le précurseur gp150 correspond à la totalité de la séquence codante du géne env, telle qu'elle est déduite de la séquence du génome viral comme déjà rappelé plus haut, c'est-à-dire pour une chaîne polypeptidique de 95K, si l'on fait abstraction des groupes carbohydrate.

Après le premier clivage protéolytique qui semble devoir prendre place soit dans le cytoplasme, soit à proximité de la membrane cellulaire, la gp150 est transformée dans la glycoprotéine gp135 (à laquelle correspond une ossature polypeptidique de 80K, après séparation des groupes carbohydrate). Au cours de la morphogénèse du virus, la gp135 est transformée à son tour dans la gp110-120, du fait de l'élimination enzymatique partielle de groupes carbohydrate, en l'absence de clivage protéolytique.

Les gp150 et gp135 peuvent encore être obtenus et purifiés à partir des lysats de syncitia correspondants, par la même technique que celle qui a été indiquée dans la demande de brevet principal, en ce qui concerne la gp110.

De même il est remarqué que les techniques de production d'anticorps, plus particulièrement d'anticorps monoclonaux, décrits dans la demande de brevet principal en rapport avec l'isolement de la gp110, peuvent être appliquées dans des conditions tout à fait semblables à la production d'anticorps, plus particulièrement d'anticorps monoclonaux spécifiques de la gp150 et de la gp135. Ces anticorps monoclonaux peuvent à leur tour, notamment après immobilisation sur un support adéquat et dans les mêmes conditions qu'évoquées dans la demande de brevet principal, en ce qui concerne la gp110, être utilisés pour les purifications plus poussées de la gp150 et de la gp135.

A l'instar de ce qui a été décrit pour la gp110 dans la demande de brevet principal, les gp150 et gp135 pourront être utilisées -dans des conditions semblables -pour la production de compositions immunogènes. En particulier l'invention concerne des compositions immunogènes contenant les antigènes conformes à la présente invention, en association avec un véhicule physiologiquement acceptable, permettant son administration à un hôte vivant, notamment l'homme. Une telle composition est par exemple caractérisée en ce qu'elle est dosée en antigène de façon à permettre l'administration de doses unitaires de 10 à 500, notamment de 50 à 100 microgrammes/kg.

Dans les mêmes conditions encore que celles évoquées à propos de la gp110 dans la demande de brevet principal, la gp150 et la gp135 pourront être mises en oeuvre dans des procédés et "kits" de diagnostic pour la détection d'anticorps anti-LAV dans un milieu biologique adéquat, notamment le sérum ou le liquide céphalo-rachidien, de patients affectés des maladies occasionnées par les virus LAV ou analogues ou de personnes immunisées contre ces virus. L'utilisation de la gp150 ou de la gp135 dans des essais ou "kits" de diagnostic peuvent s'avérer particulièrement avantageux à l'égard de ceux des sérums qui peuvent les reconnaître préférentiellement à la gp110. On se reportera encore à la description du brevet principal pour ce qui est de la description des conditions dans lesquelles ces procédés et "kits" de diagnostic peuvent être mis en oeuvre, ainsi qu'à certaines des revendications de la présente demande de certificat d'addition, lesquelles, outre qu'elles définissent des formes préférées des procédé et "kits" de diagnostic de l'invention sont à considérer comme faisant partie intégrante de la description.

Les hybrides cellulaires exprimant à leur surface la gp150, ou à la fois la gp150 et la gp135, font également partie de l'invention. Ces hybrides cellulaires peuvent eux-mêmes, particulièrement lorsqu'ils sont à l'état fixé, faire partie de "kits" de diagnostic. La méthode de diagnostic peut alors faire intervenir la mise en contact de ces hybrides cellulaires porteurs de ces glycoprotéines ou fragments de glycoprotéine et préalablement fixés par exemple par l'acétone ou toute solution de fixation adéquate avec les sérums des patients ou personnes susindiquées, puis la détection sur les hybrides éventuellement fixés, par des méthodes classiques, notamment à l'aide d'anticorps marqués de souris anti-immunoglobulines humaines - (marqueurs radioactifs, enzymatiques, fluorescents, etc.).

Dans ce qui suit, il sera fourni des informations complémentaires en ce qui concerne la gp110, ses propriétés et sa relation à d'autres antigènes susceptibles d'être isolés à partir de cultures de lymphocytes ou des cellules CEM respectivement infectées avec le virus LAV (en l'occurrence LAV1 dans le complément de description qui suit). Les cellules CEM utilisées ont résulté d'une sélection parmi la lignée ATCC CCL 19 de celles des souches (déposées le 29 janvier 1985 à la C.N.C.M. sous les n° I-416 et I-417) qui se sont révélées posséder des molécules T4 à leurs surfaces.

En particulier ces informations ont été obtenues en procédant comme suit.

Des lymphocytes T d'un donneur normal et des cellules CEM ont été infectées avec le virus LAV1, puis marqués avec la $^{35}$S-cystéine dans un milieu libre de cystéine non marquée. Le surnageant clarifié de la culture des cellules a ensuite été traité par centrifugation comme décrit dans la demande de brevet principal, ce surnageant ayant ensuite été lysé dans un tampon RIPA, puis incubé avec des sérums humains provenant de personnes infectées ou non infectées par le LAV.

Les lysats de virus, analysés comme il sera décrit dans la légende de la fig. 2, donnent à chaque fois lieu à la détection par des sérums provenant de patients affectés par le SIDA, à la reconnaissance non seulement de la gp110, mais de protéines en fines bandes dans des gels d'électrophorèse des susdits lysats correspondant respectivement à des poids moléculaires 70K, 40K et 34K, toutes spécifiquement immunoprécipitées par les sérums desdits patients. Dans la mesure où ces sérums ne reconnaissent aucune protéine gag, il peut être présumé que ces protéines sont antigéniquement apparentées à la gp110 et qu'elles en sont des produits de clivage. Ces mêmes bandes sont davantage apparentes, lorsque l'on a recours à la technique de transfert sur papier filtre dite "immunoblottine" à laquelle référence est faite dans la légende de la fig. 2.

Des résultats analogues sont obtenus lorsque des cultures de lymphocytes T infectés ont été métaboliquement marquées avec la [$^{14}$C]-glucosamine. Les lysats du virus préablablement centrifugés ont également conduits à des bandes d'immunoprécipitation avec un sérum LAV-positif dans des essais réalisés sur gel de polyacrylamide contenant du SDS (SDS-PAGE). En sus des bandes spécifiquement reconnues par lesdits sérums au niveau de la gp110, on a également observé des immunoprécipitations spécifiques au niveau de bandes 70K et 42K

Le fait que ces protéines de 70K et 40K constituent plus que vraisemblablement des produits de clivage de la protéine gp110 est encore confirmé par l'examen de la séquence nucléotidique de génome viral, qui fait apparaître un site protéolytique potentiel au niveau du deuxième tiers du gène env. Vraisemblablement la protéine 40K correspond à la portion transmembranaire de la glycoprotéine d'enveloppe.

Se référant encore à la séquence nucléotidique de l'article de WAYN-HOBSON, il paraît que les protéines 70K et 40K ont en commun des séquences polypeptidiques avec les polypeptides que codent respectivement les séquences nucléotidiques s'étendant : _

-entre la position 5767 et la position approximative 7297-7327, pour la protéine 70K et

-entre la position approximative 7297-7327 et la position 8350, pour la protéine 40K.

Les protéines 70K et 40K font donc elles-mêmes partie de l'invention. Elles peuvent elles-mêmes, tout comme les glycoprotéines gp110, gp135 et gp150, être utilisées dans les procédés et "kits" de diagnostic qui ont été décrits en rapport avec les dernières glycoprotéines.

Il va de soi que l'invention concerne de la même façon des polypeptides équivalents à ceux qui ont été plus particulièrement décrits dans cette demande de brevet (que ces polypeptides soient pourvus de groupes carbohydrate ou non), notamment des polypeptides ayant des séquences aminoacides semblables et produits par les techniques du génie génétique. Il va également de soi qu'entrent dans le cadre des revendications les polypeptides dont certaines séquences d'aminoacides seraient modifées, sans que soient modifées sensiblement les propriétées immunologiques des polypeptides en cause. A ce titre l'invention concerne les polypeptides équivalents provenant d'autres espèces de virus LAV que celui qui a plus particulièrement été mis en oeuvre dans la présente demande.

Légende de la figure 1 : Effet de l'endo-H sur la

glycoprotéine d'enveloppe du LAV et ses

précurseurs cellulaires.

Des lysats de syncitia d'hybridomes MOLT-4/T4 infectés par le LAV, marqués pendant 12 heures avec de la $^{35}$S-cystéine (colonnes a à d) et de virions LAV marqués par la $^{35}$S-cystéine et séparés par centrifugation (colonnes e à h) ont été immunoprécipités avec le sérum d'un patient souffrant de lymphadénopathie persistante (BRU). Les complexes immuns ont été liés à des perles de l'agarose commercialisée sous la marque SEPHAROSE auxquelles était liée de la protéine A, ces complexes immuns ayant ensuite été lavés à plusieurs reprises, puis élués par chauffage à 95°C pendant deux minutes dans un tampon citrate pH 5,5, contenant 0,1 % de SDS et 5 % d'aprotinine - (ZYMOFREN, SPECIA). Les antigènes solubilisés ont ensuite été traités avec 0,5 microgramme d'endo-H dans un volume final de 22 microlitres sur différentes durées à 37° C, avant d'être analysés par électropherèse sur gel de SDS-polyacrylamide (7,5 % d'acrylamide et 0,17 % de bisacrylamide).

Les observations suivantes sont à faire en ce qui concerne les différentes colonnes de gel :

-colonnes a et e : pas de traitement ;

-colonnes b et f : 1 heure de traitement ;

-colonnes c et g : 2 heures de traitement ;

-colonnes d et h : 3 heures de traitement ;

m = marquer de poids moléculaires connus.

Légende de la fig. 2: Analyse par le technique du

"Westernblot".

Des surnageants de cellules infectées par le LAV, également de cellules infectées par HTLV-3 ont été concentrés par ultracentrifugation. Les culots de centrifugation obtenus à partir de préparations virales et de préparations témoins - (ces culots ayant des concentrations totales en protéines de 5 à 10 grammes) ont été fractionnés sur un gel de polyacrylamide à 12 % contenant du SDS, puis transférés électrophorétiquement sur feuilles de nitrocellulose, puis transférés selon la technique de l'immunotransfert (immunoblot) comme décrit par PAREKH B., et al, Virology, 107, 520 (1980). Le papier filtre comprenant les bandes a ensuite été incubé avec le sérum d'un patient atteint de LAS (dilué au 1/200°), lavé, incubé avec des IgG anti-humaines de chèvre, couplées avec de la péroxydase, et lavé à nouveau. La détection des anticorps a été effectuée en utilisant en tant que substrat de la diamino-benzidine en tant que substrat pour la réaction péroxydase. Des observations semblables ont été faites avec les lysats de virus obtenus à partir de cellules H9 infectées avec le LAV ou le virus HTLV-III.

Les observations suivantes résultent de l'examen de la fig. 4 :

-colonne 1 : culot viral provenant de cellules H9 infectées avec le LAV;

-colonne 2 : culot viral obtenu à partir de cellules H9 infectées avec le HTLV-III:

-collone 3 : culot viral obtenu à partir de cellules·CEM produisant du LAV;

-colonne 4 : culot viral obtenu à partir de cellulose H9 non infectées;

-les flèches identifient de haut en bas les bandes correspondent aux glycoprotéines ou protéines suivantes : gp110, p70, p40, p34, p25 et p18.

## Revendications

1 -Antigène caractérisé par l'ensemble des propriétés suivantes:

-il possède un poids moléculaire de l'ordre de 150.000,

-il possède une ossature polypeptidique comportant une séquence polypeptidique en commun avec la glycoprotéine d'enveloppe du virus LAV.

-il est reconnu par des sérums de patients affectés par un SIDA ou un LAS dû au virus LAV ou par des sérums de porteurs asymptomatiques du virus LAV.

-il a la capacité d'être marqué par la $^{35}$S-cystéine;

-il a la capacité de former des complexes avec la concanavaline A, ces complexes pouvant ensuite être dissociés en présence de O-méthyl-alpha-D-mannapyranoside;

-il a la capacité de former des complexes avec d'autres lectines, telles que celles connues sous la désignation "LENTYL-LECTINES";

-il est sensible à l'action d'endoglycosidases, particulièrement de l'endo-bêta-N-méthylglycosaminidase-H, cette action s'accompagnant d'une formation de protéines ayant un poids moléculaire d'environ 95.00;

-il a la capacité d'être marqué par la [$^{14}$C]-glucosamine.

2 -Antigène selon la revendications 1, caractérisé en ce qu'il possède une ossature peptidique correspondant à celle codée par la totalité du gène env du virus LAV.

3 - Antigène caractérisé par l'ensemble des propriétés suivantes:

-il possède un poids moléculaire de l'ordre de 135.000,

-il possède une ossature polypeptidique comportant une séquence polypeptidique en commun avec la glycoprotéine d'enveloppe du virus LAV,

-il est reconnu par des sérums de patients affectés par un SIDA ou un LAS dû au virus LAV ou par des sérums de porteurs asymptomatiques du virus LAV.

-il a la capacité d'être marqué par la $^{35}$S-cystéine;

-il a la capacité de former des complexes avec la concanavaline A, ces complexes pouvant ensuite être dissociés en présence de O-méthyl-alpha-D-mannopyranoside;

-il a la capacité de former des complexes avec d'autres lectines, telles que celles connues sous la désignation "LENTYL-LECTINES";

-il est sensible à l'action d'endoglycosidases, particulièrement de l'endo-bêta-N-méthylglycosaminidase-H, cette action s'accompagnant d'une formation de protéines ayant'un poids moléculaire d'environ 80.000;

-il a la capacité d'être marqué par la ["¹⁴C]-glucosamine.

4 -Antigène selon la revendication 3, caractérisé en ce que son ossature peptidique est dérivée de l'ossature peptidique totale codée par la totalité du gène env du virus LAV, cependant dépourvue d'une séquence peptidique ayant un poids moléculaire d'environ 15.000 et correspondant à la partie C-terminale de la susdite ossature totale.

5 -Procédé d'obtention d'un antigène conforme à l'une quelconque des revendications 1 à 4, comprenant l'infection d'une lignée de cellules hybrides exprimant la molécule T4 à leur surface et étant susceptible à l'infection par le virus LAV, ces cellules hybrides ayant préalablement été formées par fusion de lymphocytes humains T4 et de cellules lymphoïdes tumorales, notamment du type MOLT-4, la culture des cellules infectées de ces lignées hybrides, la récupération et la lyse des cellules hybrides infectées, la séparation des antigènes produits et la récupération, selon le cas, de la glycoprotéine ayant un poids moléculaire de l'ordre de 150.000 ou de la glycoprotéine ayant un poids moléculaire de l'ordre de 135.000 ou de l'une et de l'autre de ces glycoprotéines.

6 -Procédé selon la revendication 5, caractérisé en ce que la lyse des cellules hybrides infectées est effectuée avant la fin du processus infectieux qui s'achève avec une production de particules virales libres nouvelles.

7 -Procédé selon la revendication 6, caractérisé en ce que la lyse est réalisée 3 heures après l'infection et en ce que l'on récupère la glycoprotéine ayant un poids moléculaire d'environ 150.000.

8 -Procédé selon la revendication 6, caractérisé en ce que la lyse est ralisée 12 heures après l'infection et en ce que l'on récupère la glycoprotéine ayant un poids moléculaire d'environ 135.000.

9 - Procédé selon l'une quelconque des revendications 5 à 8, caractérisé en ce que la séparation desdits antigènes comprend la mise en contact du milieu contenant lesdits antigènes avec une lectine affine, telle que le concanavaline A ou la lentyl-lectine, cette lectine étant de préférence immobilisée sur un support solide, puis l'élution du susdit antigène, notamment à l'aide d'une solution de O-méthyl-alpha-D-mannopyranoside.

10 -Procédé selon la revendication 9, caractérisé en ce que ladite purification est réalisée par immunoprécipitation par les sérums de patients connus pour posséder des anticorps actifs contre ledit antigène ou encore avec des anticorps monoclonaux produits par des hybridomes préalablement formés et secrétant lesdits anticorps monoclonaux, ceux-ci reconnaissant plus particulièrement, selon les cas, soit l'antigène ayant un poids moléculaire d'environ 150.000, soit l'antigène ayant un poids moléculaire d'environ 135.000.

11 -Anticorps monoclonaux caractérisés par leurs capacités à reconnaître spécifiquement l'antigène conforme à la revendication 1 ou, selon le cas, l'antigène conforme à la revendication 3.

12 -Les hybridomes secréteurs des anticorps monoclonaux selon la revendication 11.

13 -Lignée de cellules hybrides formées entre des lymphocytes T4 et des cellules du type de celles de la lignée MOLT-4, les cellules hybrides desdites lignées étant caractérisées par leur capacité à exprimer la moléculaire T4 à leur surface et par leur susceptibilité à l'infection par le virus LAV ou par des virus apparentes.

14 -Lignée de cellules hybrides selon la revendication 12, caractérisée en ce qu'elle est identique à la lignée déposée le 15 avril 1985 à la C.N.C.M. sous le n° I-435 ou en est dérivée.

15 -Lignée de cellules hybrides selon la revendication 13 ou la revendication 14, caractérisée en ce qu'elles portent à leur surface un antigène conforme à l'une quelconque des revendications 1 à 4 et en ce que les cellules de cette lignée sont fixées.

16 -Procédé de détection in vitro de la présence d'anticorps anti-LAV dans un fluide biologique humain, tel que sérum ou liquide céphalorachidien, comprenant la mise en contact de ce liquide biologique avec un antigène conforme à l'une des revendications 1 à 4 et la détection du complexe antigène-anticorps formé.

17 -Procédé selon la revendication 16, caractérisé en ce que l'antigène est exposé à la surface de cellules infectées et fixées.

18 -Procédé selon la revendication 16, caractérisé en ce que l'antigène est fixé à la surface d'un support solide.

19 -Procédé de diagnostic de la présence d'anticorps antivirus LAV dans un sérum ou milieu biologique équivalent, comprenant :

- le dépôt de quantités déterminées d'un antigène selon l'une quelconque des revendications 1 à 4 dans les puits d'une microplaque de titrage ;

-l'introduction dans ces puits de dilutions croissantes de sérum à diagnostiquer ;

-l'incubation de la microplaque ;

-le lavage soigneux de la microplaque ;

-l'introduction dans les puits de la microplaque d'anticorps marqués spécifiques d'immunoglobulines du sang, le marquage étant réalisé par une enzyme choisie parmi celles qui sont capables d'hydrolyser un substrat de telle sorte que ce dernier subit alors une modification de son absorption des radiations, au moins dans une bande de longueurs d'ondes déterminée et

-la détection, de préférence de façon comparative par rapport à un témoin, de l'importance de l'hydrolyse du substrat en tant que mesure des risques potentiels ou de la présence effective de la maladie.

20 -Nécessaire ou "kit" de diagnostic de la présence d'anticorps antivirus LAV dans un échantillon biologique approprié, notamment un sérum humain, caractérisé par le fait qu'il comporte entre autres réactifs usuels, notamment pour l'analyse selon la technique ELISA, un antigène conforme à l'une quelconque des revendications 1 à 4, cet antigène étant retenu à la surface d'un support ou exposé à la surface de cellules hybrides infectées et fixées.

21 -Composition immunogène caractérisée par l'association d'un antigène conforme à l'une quelconque des revendications 1 à 4, avec un excipient physiologiquement acceptable, permettant son administration à un hôte vivant, plus particulièrement l'homme.

22 -Composition immunogène selon la revendication 13, caractérisée en ce qu'elle est dosée en antigène de façon à permettre l'administration d'une dose de 10 à 500, notamment de 50 à 100 microgrammes par kilogramme.

# FIG.1

a   b   c   d   e   f   g   h   m

-200

150
135
110
95
80

-93

-69

-46

# FIG.2

1 2 3 4

110 →

68 →

40 →

34 →

25 →

18 →

# RAPPORT DE RECHERCHE EUROPEENNE

Office européen des brevets

Numéro de la demande

EP 86 40 0797

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 103, no. 5, 5 août 1985, page 263, résumé no. 34641v, Columbus, Ohio, US; L. MONTAGNIER et al.: "Identification and antigenicity of the major envelope glycoprotein of lymphadenopathy-associated virus", & VIROLOGY, 1985, 144(1), 283-289 * En entier * | 1-4 | C 07 K 15/00<br>C 12 P 21/00<br>C 12 N 5/00<br>C 12 N 15/00<br>C 12 N 7/00<br>C 07 K 3/18<br>G 01 N 33/569<br>A 61 K 39/21 //<br>(C 12 P 21/00<br>C 12 R 1:91 ) |
| A | SCIENCE, vol. 224, no. 4648, 4 mai 1984, pages 503-505; J. SCHÜPBACH et al.: "Serological analysis of a subgroup of human T-lymphotropic retroviruses (HTLV-III) associated with AIDS" * Résumé, page 503; page 505, lignes 37-41 * | 1-4 | |
| A | NATURE, vol. 313, 7 février 1985, pages 450-458; M.A. MUESING et al.: "Nucleic acid structure and expression of the human AIDS/lymphadenopathy retrovirus" * Page 454, ligne 67 - page 455, colonne de droite, ligne 15; page 457, colonne de droite, lignes 5-25 * | 1-4 | A 61 K<br>C 12 P<br>G 01 N |

DOMAINES TECHNIQUES RECHERCHES (Int. Cl.4)

--- -/-

Le présent rapport de recherche a été établi pour toutes les revendications

| Lieu de la recherche | Date d'achèvement de la recherche | Examinateur |
|---|---|---|
| LA HAYE | 25-07-1986 | CHARLES D.J.P.I.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82

## DOCUMENTS CONSIDERÉS COMME PERTINENTS

Page 2

| Catégorie | Citation du document avec indication, en cas de besoin, des parties pertinentes | Revendication concernée | CLASSEMENT DE LA DEMANDE (Int. Cl.4) |
|---|---|---|---|
| A | NATURE, vol. 312, 20/27 décembre 1984, pages 763-767; A.G. DALGLEISH et al.: "The CD4 (T4) antigen is an essential component of the receptor for the AIDS retrovirus" * Page 763, colonne de droite, résumé; page 764, colonne de droite, lignes 7-22 * | 5,13-15 | |
| A | WO-A-8 404 327 (PRESIDENT AND FELLOWS OF HARVARD COLLEGE) * Page 2, lignes 14-32; page 4, ligne 11 - page 6, ligne 9; page 6, ligne 28 - page 7, ligne 1; page 7, ligne 23 - page 8, ligne 14; page 13, ligne 1 - page 14, ligne 15; revendications * | 16-20 | |
| A | EP-A-0 136 798 (BIOTECH RESEARCH LABORATORIES INC.) * Revendications * | 16-20 | DOMAINES TECHNIQUES RECHERCHES (Int. Cl.4) |
| A | BIOLOGICAL ABSTRACTS, vol. 79, 1985, réf. no. 105818, Biological Abstracts Inc., Philadelphia, US; J.S. McDOUGAL et al.: "Immunoassay for the detection and quantitation of infectious human retrovirus. Lymphadenopathy-associated virus", & J. UMMUNOL. METHODS 76(1), 171-184, 1985 * En entier * | 16-20 | |

Le présent rapport de recherche a été établi pour toutes les revendications

| Lieu de la recherche | Date d'achèvement de la recherche | Examinateur |
|---|---|---|
| LA HAYE | 25-07-1986 | CHARLES D.J.P.I.G. |